# EUROPEAN PATENT APPLICATION

(11) **EP 2 340 832 A1**
(43) Date of publication of application: **06.07.2011**
(21) Application number: 09015938.5
(22) Date of filing: 23.12.2009
(51) Int. Cl.: A61K 31/485, A61P 25/16, A61P 25/28

(54) **Morphinan-6-one compounds for the treatment or prevention of neurodegenerative diseases**

(71) Applicant: Universität Innsbruck, 6020 Innsbruck (AT)
(72) Inventor: Rollinger, Judith M., 6020 Innsbruck (AT); Prast, Helmut, 6020 Innsbruck (AT); Schuster, Daniela, 6020 Innsbruck (AT); Langer, Thierry, 6175 Ranggen (AT); Hornick, Ariane, 6020 Innsbruck (AT); Stuppner, Hermann, 6091 Götzens (AT)
(74) Representative: Schrell, Andreas

(57) **Abstract**

The present invention relates to morphinan-6-one compounds, preferably N-formylmorphinan-6-one compounds for the treatment or prevention of neurodegenerative diseases as well as to pharmaceutical compositions, comprising at least one morphinan-6-one compound, preferably at least one N-formylmorphinan-6-one compound.

## Description

The present invention relates to morphinan-6-one compounds, preferably N-formylmorphinan-6-one compounds for the treatment or prevention of neurodegenerative diseases as well as to pharmaceutical compositions, comprising at least one morphinan-6-one compound, preferably at least one N-formylmorphinan-6-one compound.

In a society with a continuous growth of the population of over 65 years of age, a dramatic increase of neurodegenerative diseases, such as dementia or Alzheimer's disease is observed. The age-specific prevalence of dementia is approximately 1.5 % in persons aged 60 to 69 years and almost doubles every five years until it reaches 40 % in nonagenarians. One consequence of the rapid increase in the aging population world wide is that the estimated number of 24 million people that are currently affected by dementia is projected to double every 20 years. The most common manifestation of dementia, Alzheimer's disease (AD), accounts for 50 to 70 % of all dementia cases. This makes Alzheimer's disease a leading cause of mortality among the elderly. Characteristic symptoms include declination of intellectual functions (memory, language, visiospacial skills and problem solving skills) and decreasing abstract reasoning along with abnormal behaviours. Alzheimer's disease results in eventual loss of motor function, inanition and death. As a precursor to being diagnosed with Alzheimer's disease, many patients first experience mild cognitive impairment (MCI), which is thought to be a transition stage between normal aging and Alzheimer's disease or a preclinical stage of Alzheimer's disease. When memory loss is the predominant feature, this type of impairment is referred to as amnestic MCI and is likely to convert to Alzheimer's disease over time as cognitive decline increases. Alzheimer's disease is grouped into several stages. The first stage is characterized by retrospective analysis once symptoms of Alzheimer's disease have progressed, leading to the final stage consisting of dementia and severe cognitive function declination, often requiring full-time home care for the patient resulting in enormous health care expenditures.

The main cause of the loss of cognitive functions in AD patients is a continuous decline of the cholinergic neurotransmission in cortical and other regions of the human brain. At the molecular level, cholinergic neurotransmission is mediated by the neurotransmitter acetylcholine (ACh) which is rapidly hydrolyzed after its presynaptic release by acetylcholinesterase (AChE). Besides its well-known role in terminating synaptic transmission, AChE has been found to be involved in a number of other functions. For example, AChE is a factor in neurite growth and in accelerating the assembly of β-amyloid into amyloid fibrils which are characteristically found in the brain cells of AD patients. A therapy with AChE inhibitors leads to a symptomatic amelioration of memory, cognition, mood and daily living skills. Numerous studies confirmed the vital role of this medication corresponding to the "cholinergic hypothesis of learning". AChE inhibition is an approved strategy to raise ACh concentration, thereby increasing the cholinergic function in the brain. So far, four AChE inhibitors have been approved by the European and US regulatory authorities for mild neurodegenerative diseases: tacrine, donepezil, galantamine, and rivastigmine. Nonetheless, there is a need for novel, alternative medicaments with AChE inhibitor properties for the treatment or prevention of neurodegenerative diseases.

Accordingly, the technical problem underlying the present invention is to provide further efficient means for the treatment or prevention of neurodegenerative diseases, in particular compounds able to enhance cholinergic transmission, for example by acting as AChE inhibitors, in particular compounds of the known morphinan class, which are useful for the treatment or prevention of neurodegenerative diseases, and which, however, should not display agonistic properties at opioid receptors.

The present invention solves its underlying problem according to the teaching of the independent claims. Accordingly, the present invention provides in a particularly preferred embodiment morphinan-6-one compounds, preferably *N*-formylmorphinan-6-one compounds, for the treatment or prevention of neurodegenerative diseases.

Surprisingly, it could be shown that the morphinan-6-one compounds, preferably the N-formylmorphinan-6-one compounds, according to the present invention can function inter alia as enhancers of cholinergic transmission, partly by AChE inhibition, and do not display agonistic properties at opioid receptors. In fact, many compounds of the morphinan class do display such agonistic properties as they are widely used as potent analgesics. Even if these compounds possess AChE inhibitory properties, the known side effects of opioid receptor agonists render such compounds unsuitable as enhancers of cognition and memory in the treatment or prevention of neurodegenerative diseases. Therefore, the morphinan-6-one compounds, preferably the N-formylmorphinan-6-one compounds according to the present invention which surprisingly do not display agonistic properties at opioid receptors are particularly suitable for the treatment or prevention of neurodegenerative diseases. In fact, it could be shown that the compounds according to the present invention either do not display any binding affinity or they act as antagonists to opioid receptors µ, δ and K. Furthermore, they displayed no binding affinity to sigma receptors. Functional experiments, wherein the morphinan-6-one compounds, preferably the *N*-formylmorphinan-6-one compounds according to the present invention were able to increase extracellular ACh in the brain and to abolish induced cholinergic memory impairment in mice, further underlined their suitability for the treatment or prevention of neurodegenerative diseases.

In the context of the present invention the neurodegenerative diseases to be treated or prevented with the morphinan-6-one compounds, preferably the N-formylmorphinan-6-one compounds, according to the present invention can preferably be selected from, but are not limited to, the group consisting of Alzheimer's disease, Mild Cognitive Impairment, amyloidosis, Parkinson's disease, multiple sclerosis, amyotrophic lateral sclerosis, Huntington's disease, Lewy body dementia, Pick's disease, bovine spongiform encephalitis and Creutzfeldt-Jakob disease.

The terms "treatment" or "prevention" as used herein, refer to eliciting a desired pharmacological effect and/or physiologic effect in a patient, preferably human or animal patient. These effects are prophylactic or preventive in terms of completely or partially preventing a disease and/or its symptoms, and therapeutic in terms of partially or completely curing a disease and/or to alleviate or ameliorate symptoms of a disease.

In a particularly preferred embodiment of the present invention the N-formylmorphinan-6-one compound is 1,4-dihydroxy-N-formyl-morphinan-6-one of the formula:

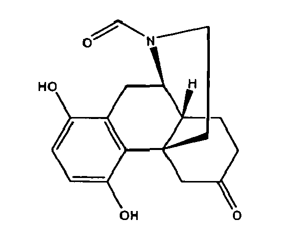

Surprisingly, it could be shown that 1,4-dihydroxy-/\/- formylmorphinan-6-one does not bind to opioid receptors µ, δ or K.

In another particularly preferred embodiment of the present invention the *N*-formylmorphinan-6-one compound is 1,4-dione-N-formyl-morphinan-6-one of the formula:

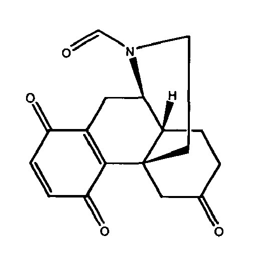

Surprisingly, it could be shown that 1,4-dione-*N*-formylmorphinan-6-one does not bind to opioid receptors µ, δ or K.

In yet another particularly preferred embodiment of the present invention the N-formylmorphinan-6-one compound is 4,5-epoxy-/\/- formylmorphinan-6-one of the formula:

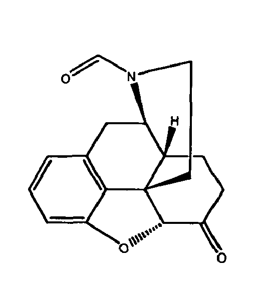

Surprisingly, it could be shown that 4,5-epoxy-*N*-formylmorphinan-6-one does not bind to opioid receptors µ, δ or K.

In a preferred embodiment of the present invention the morphinan-6-one compound is 4-methoxy-*N-*allylmorphinan-6-one of the formula:

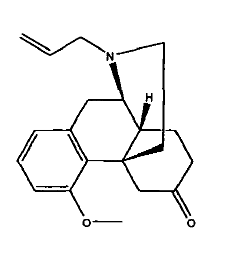

Surprisingly, it could be shown that 4-methoxy-N-allyl-morphinan-6-one has an antagonistic function at the µ opioid receptor.

In another preferred embodiment of the present invention the morphinan-6-one compound is 4,14-dimethoxy-*N*-allyl-morphinan-6-one of the formula:

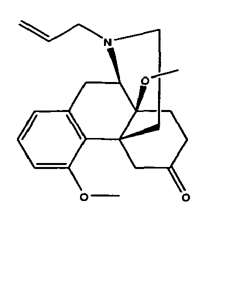

Surprisingly, it could be shown that 4,14-dimethoxy-N-allyl-morphinan-6-one has an antagonistic function at the µ opioid receptor.

The synthesis of the morphinan-6-one compounds, preferably the N-formylmorphinan-6-one compounds according to the present invention is described in Schmidhammer et al., 1981 (Heterocycles, 1981; 16: 1859-62), Schmidhammer and Brossi, 1983 (J. Org. Chem., 1983; 48: 1469-71), Schmidhammer et al., 1990 (J. Med. Chem., 1990; 33: 1200-6), Schmidhammer et al., 1990 (Progr Clin Biol Res, 328: 37-40) and Rozwadowska et al. 1980 (Can. J. Chem., 1980; 58: 1855-9) as well as US 4388463, WO 1995/031463, WO 1995/031464 and WO 2004/005294, all of these documents are incorporated herein by reference and their teaching, in particular with respect to the preparation of the morphinan-6-one compounds of the present invention is suitable and applicable for the present invention and encompassed thereby.

The morphinan-6-one compounds, in particular the N-formylmorphinan-6-one compounds, according to the present invention are preferably used, alone or in combination, for the preparation of a pharmaceutical composition for the treatment or prevention of neurodegenerative diseases in mammals, most preferably humans.

In a preferred embodiment the morphinan-6-one compounds of the present invention can be administered alone. In another preferred embodiment, it is envisaged to formulate them into a pharmaceutical composition in accordance with standard pharmaceutical practice. Thus, the invention also provides a pharmaceutical composition which comprises the morphinan-6-one compounds, in particular the *N-*formylmorphinan-6-one compounds of the present invention in admixture with a pharmaceutically acceptable excipient.

Pharmaceutically acceptable excipients are well known in the pharmaceutical art. The pharmaceutical excipient is preferably selected regarding the intended route of administration or standard pharmaceutical practice. The excipient is pharmaceutically acceptable in the sense of not being deleterious to the recipient thereof.

Pharmaceutically useful and acceptable excipients that may be used in the formulation of the pharmaceutical composition of the present invention may comprise, for example, carriers, vehicles, diluents, solvents like monohydric alcohols such as ethanol, isopropanol and polyhydric alcohols such as glycols, edible oils such as soybean oil, coconut oil, olive oil, safflower oil, cottonseed oil, oily esters like ethyl oleate, isopropyl myristate, binders, adjuvants, solubilizers, thickening agents, stabilizers, disintegrants, glidants, lubricating agents, buffering agents, emulsifiers, wetting agents, suspending agents, further additives like sweetening agents, colorants, flavors, coating agents, preservatives, antioxidants, processing agents, drug delivery modifiers and enhancers such as calcium phosphate, magnesium state, talc, monosaccharides, disaccharides, starch, gelatine, cellulose, methylcellulose, sodium carboxymethyl cellulose, dextrose, hydroxypropyl-β-cyclodextrin, polyvinylpyrrolidone, low melting waxes, and ion exchange resins.

The routes for administration or delivery of the morphinan-6-one compounds of the present invention include, but are not limited to, a topical or systemic administration, in particular are one or more of: oral, for instance in form of a tablet, capsule, or as an ingestible solution, topical, parenteral, for instance in an injectable form, subcutaneous, intramuscular, intravenous, gastrointestinal, intraspinal, intracerebral, intracranial, intracerebroventricular, intraperitoneal, intraocular, intradermal, intratracheal, intrauterine, intravaginal, ophthalmic, including intravitreal or intracameral, transdermal, mucosal for instance as a nasal spray or aerosol for inhalation, rectal, buccal, epidural and sublingual.

For example, the morphinan-6-one compounds according to the present invention can be administered orally in the form of tablets, capsules, elixirs, solutions or suspensions, which may also contain flavouring or colouring agents, formulated for immediate-, delayed-, modified-, sustained-, pulsed- or controlled-release applications.

Oral formulations like tablets preferably contain pharmaceutically acceptable excipients such as microcrystalline cellulose, lactose, sodium citrate, calcium carbonate, dibasic calcium phosphate or glycine, disintegrants such as starch (preferably corn, potato or tapioca starch), sodium starch glycollate, croscarmellose sodium or certain complex silicates, and granulation binders such as polyvinylpyrrolidone, hydroxypropylmethylcellulose (HPMC), hydroxypropylcellulose (HPC), sucrose, gelatine or acacia. Additionally, lubricating agents such as magnesium stearate, stearic acid, glyceryl behenate and talc may be used. Solid compositions of a similar type may also be employed as fillers in gelatine capsules. Preferred excipients in this regard include lactose, starch, cellulose, milk sugar or high molecular weight polyethylene glycols. For aqueous suspensions and/or elixirs, the morphinan-6-one compounds of the present invention may be combined with various sweetening or flavouring agents, colouring matters or dyes, emulsifying and/or suspending agents or with diluents such as water, ethanol, propylene glycol and glycerine, and combinations thereof.

If the morphinan-6-one compounds according the present invention are administered parenterally, such administrations may include one or more of: intravenously, intraarterially, intramuscularly, subcutaneously, intraperitoneally, intrathecally, intraventricularly, intraurethrally, intrasternally, intracranially, or the compounds are administered by using infusion techniques. For parenteral administration, the compounds are most suitably used in the form of a sterile aqueous solution which may contain other substances, for example, enough salts or glucose to make the solution isotonic with blood. The aqueous solutions should be suitably buffered, preferably to a pH of from 3.0 to 9.0, if necessary. The preparation of such parenteral formulations under sterile conditions is readily accomplished by standard pharmaceutical techniques well known to those skilled in the art.

The morphinan-6-one compounds according the present invention may also be administered intranasally or by inhalation and are conveniently delivered in the form of a dry powder inhaler or an aerosol spray presentation from a pressurized container, pump, or nebulizer using a suitable propellant, for instance dichlorodifluoromethane, trichlorofluoromethane, dichlorotetrafluoroethane, a hydrofluoroal-kane such as 1,1,1,2-tetrafluoroethane (HFAI 34AT) or 1,1,1,2,3,3,3-heptafluoropropane (HFA 227EA) or carbon dioxide. In the case of a pressurized aerosol, the dosage unit might be determined by a valve delivering a metered amount. The pressurized container, pump, or nebulizer may contain a solution or suspension of the active compound of the present invention, such as a mixture of ethanol and a propellant as the solvent, which may additionally contain a lubricant, e. g. sorbitan trioleate. Capsules, for example prepared from gelatine, for use in an inhaler, may be formulated to contain a powder mix of the compounds of the present invention and a suitable powder base such as lactose or starch.

In another preferred embodiment, the morphinan-6-one compounds of the present invention can be administered in the form of a suppository or pessary, or may be applied topically in the form of a gel, hydrogel, lotion, solution, cream, ointment or dusting powder. The compounds of the present invention may also be administered dermally or transdermally, for example, by the use of a skin patch.

The morphinan-6-one compounds of the present invention may also be administered by the ocular route. For ophthalmic use, the compounds can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may also be formulated in form of an ointment such as petrolatum.

For topical application to the skin, the morphinan-6-one compounds according the present invention can be formulated as an ointment containing the active compound suspended or dissolved, for example, in a mixture with one or more of the following components: mineral oil, liquid petrolatum, white petrolatum, propylene glycol, emulsifying wax or water. Alternatively, they may also be formulated as a lotion or cream, suspended or dissolved, for example, in a mixture of one or more of the following components: mineral oil, sorbitan monostearate, polyethylene glycol, liquid paraffin, polysorbate 60, cetyl esters, wax, cetearyl alcohol, 2-octyldodecanol, benzyl alcohol or water.

The specific dose as well as the frequency of dosage for any particular subject or patient may vary and depends on a variety of factors including the activity of the specific morphinan-6-one compound according to the invention that is employed, the metabolic stability and length of action of that compound, the age, body weight, general health, sex, diet, mode and time of administration, rate of excretion, drug combination, the severity of the particular condition, and the subject or patient undergoing therapy.

A proposed dosage of the morphinan-6-one compounds according to the present invention for administration to a human (of approximately 70 kg body weight) is 0.1 mg to 1 g, preferably 1 mg to 500 mg of the active ingredient per dose unit. The dose unit may be administered, for example, 1 to 4 times per day. The dosage depends on the route of administration.

Thus, a particularly preferred embodiment of the present invention is a pharmaceutical composition comprising at least one morphinan-6-one compound, in particular a N-formylmorphinan-6-one compound, according to the present invention and at least one pharmaceutically acceptable excipient for the treatment or prevention of neurodegenerative diseases.

In a particularly preferred embodiment of the present invention the pharmaceutical composition according to the present invention comprises at least the morphinan-6-one compound, in particular the N-formylmorphinan-6-one compound, of the present invention, optionally one pharmaceutically acceptable excipient or carrier and, in a preferred embodiment, a written instruction, for instance in form of a package or an application or instruction leaflet, all of them providing administration and/or dosis instructions.

Thus, the present invention also relates to a kit comprising at least one morphinan-6-one compound, preferably an N-formylmorphinan-6-one compound, of the present invention, optionally together with a pharmaceutical acceptable carrier accompanied by written instructions, preferably on an application or instruction leaflet or a package. In a particularly preferred embodiment, the pharmaceutical composition of the present invention, in particular the morphinan-6-one compound of the present invention, is specifically adapted and prepared for its intended use, i. e. the treatment or prevention of neurodegenerative diseases. In a preferred embodiment of the present invention said preparation and adaption for the intended use is realised by written instructions on for instance an instruction or application leaflet or a package including administration and/or dosis instructions accompanying the pharmaceutical composition or the compound of the present invention.

In a preferred embodiment the present invention relates to a pharmaceutical composition comprising one or more morphinan-6-one compounds according to the present invention and optionally at least one further biologically active compound and/or a pharmaceutically acceptable carrier, diluent or excipient. The further biologically active substance can be a known compound used in the medication of neurodegenerative diseases and disorders such as Alzheimer's disease.

The further biologically active substance or compound may exert its biological effect by the same or a similar mechanism as the compound according to the invention or by another mechanism of action or by a multitude of related and/or unrelated mechanisms of action.

The morphinan-6-one compounds according to the present invention can also be provided in the form of a mixture with at least one further biologically active compound and/or a pharmaceutically acceptable carrier and/or a diluent and/or an excipient. The compound and/or the further biologically active compound are preferably present in a therapeutically effective amount.

In a preferred embodiment, the further biologically active compound may include neural-transmission enhancers, psychotherapeutic drugs, cholesterol-lowering agents, acetylcholinesterase inhibitors such as tacrine, rivastigmine, donepezil, and/or galantamine, calcium-channel blockers, NMDA receptor antagonists, biogenic amines, benzodiazepine tranquillizers, acetylcholine synthesis, storage or release enhancers, acetylcholine postsynaptic receptor agonists, monoamine oxidase-A or -B inhibitors, N-methyl-D-aspartate glutamate receptor antagonists, non-steroidal anti-inflammatory drugs, antioxidants, or serotonergic receptor antagonists. In particular, the further biologically active compound can be selected from the group consisting of compounds used in the treatment of neurodegenerative diseases such as amyloidoses, compounds against oxidative stress, anti-apoptotic compounds, metal chelators, inhibitors of DNA repair such as pirenzepin and metabolites, 3-amino-1-propanesulfonic acid (3APS), 1,3-propanedisulfonate (1,3PDS), α-secretase activators, β- and γ-secretase inhibitors, tau proteins, neurotransmitters, β-sheet breakers, attractants for amyloid beta clearing or depleting, inhibitors of N-terminal truncated amyloid beta including pyroglutamated amyloid beta 3-42, Ml agonists, or other drugs including any amyloid or tau modifying drug and nutritive supplements, an antibody or functional parts thereof, an Amyloid-β antigenic peptide fragment consisting of a single or repetitive stretch of a plurality of contiguous amino acid residues from the N-terminal part of the Amyloid-β peptide.

In a particularly preferred embodiment of the present invention, the mixtures according to the present invention may comprise niacin or memantine together with a compound according to the present invention and, optionally, a pharmaceutically acceptable carrier and/or a diluent and/or an excipient.

In another preferred embodiment of the present invention, mixtures are provided that comprise as a further biologically active compound atypical antipsychotics such as clozapine, ziprasidone, risperidone, aripiprazole or olanzapine for the treatment of psychotic symptoms including hallucinations, delusions, thought disorders, e. g. manifested by marked incoherence, derailment, tangentiality, and strange or disorganized behavior, as well as anhedonia, flattened affect, apathy, or social withdrawal, together with a compound according to the invention and, optionally, a pharmaceutically acceptable carrier, diluent or excipient.

Thus, the compounds of the invention may also be used in combination with other therapeutic agents. When a compound of the invention is used in combination with a second therapeutic agent, active against the same disease, the dose of each compound may differ from when the compound is used alone.

Combinations of the morphinan-6-one compound of the present invention with other therapeutic agents may conveniently be presented in form of a pharmaceutical formulation. The individual compounds of such combinations may be administered either sequentially or simultaneously in separate or combined pharmaceutical formulations by any convenient route of administration. When administration is sequential, either the morphinan-6-one compound of the present invention or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in formulations convenient for such compounds.

The pharmaceutical compositions of the present invention can be produced in a manner known to the skilled person or as described, for example, in Remington's Pharmaceutical Sciences, 15th Ed., Mack Publishing Co., New Jersey (1991).

The morphinan-6-one compounds, in particular the N-formylmorphinan-6-one compounds according to the present invention can also be conveniently administered or prepared, alone or in combination, as active or nutritional ingredients in food products, functional food or as dietary supplements.

In a furthermore preferred embodiment the present invention relates to a method of treatment or prevention of a neurodegenerative disease, wherein an animal or human patient in need thereof is subjected to a preventive administration or treatment with a suitable dose of the morphinan-6-one compound of the present invention for a suitable period of time, in particular with a pharmaceutical composition comprising such a suitable dose of the morphinan-6-one compounds, in particular the N-formylmorphinan-6-one compounds of the present invention for a suitable period of time, to prevent or cure the neurodegenerative disease.

Further preferred embodiments are the subject matter of the subclaims.

The invention is further illustrated by the following non-limiting examples and the accompanying figures.

The figures show:

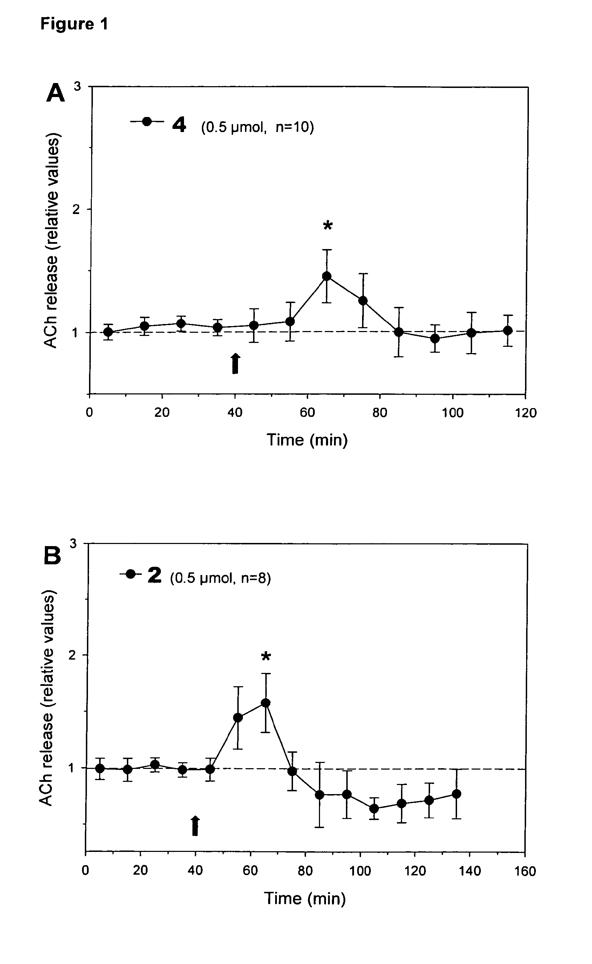

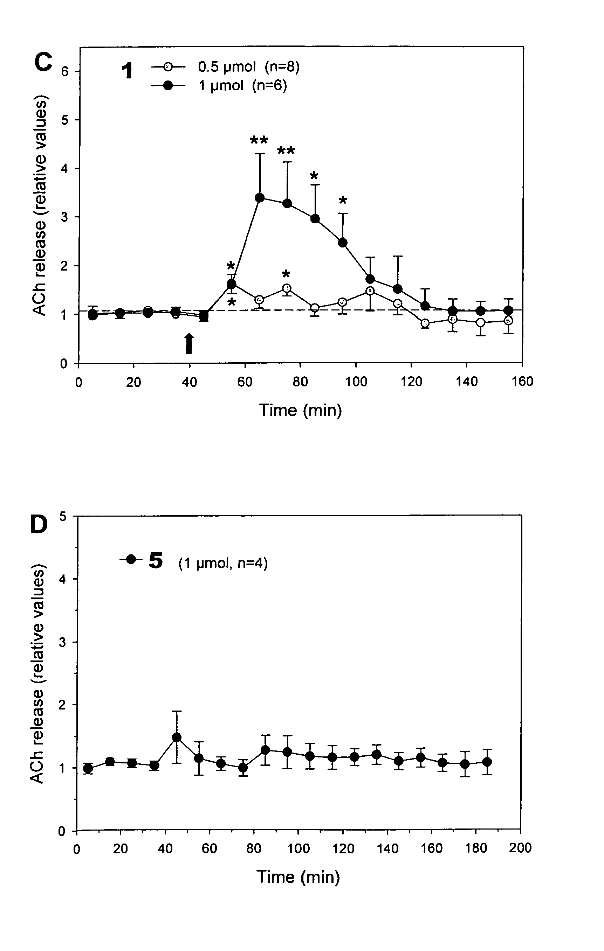

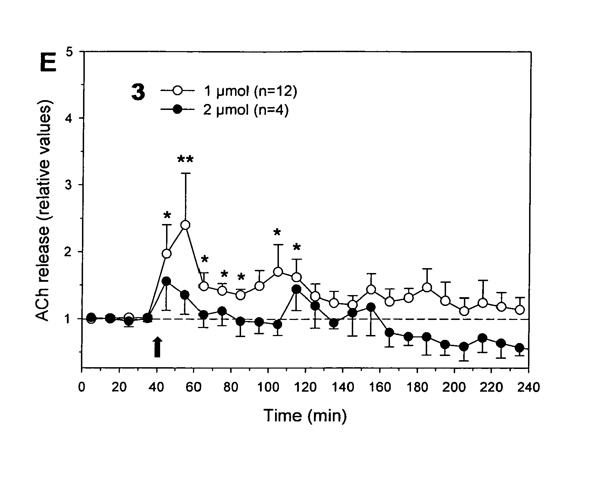

Fig. 1 shows the effect of the morphinan-6-one compounds on extracellular ACh concentration in the nucleus accumbens. A: 4, B: 2, C: 1, D: 5, E: 3. Arrows indicate the time when i.c.v. injection (10 µl at 2 µl/min) of compound solutions was started. Data are shown as relative values of basal release which is the mean of four pre-injection samples. Basal release is taken as 1.0 and indicated by a dotted line. **p* < 0.05, Friedman's analysis of variance followed by Dunn's multiple comparisons test using the pre-injection sample as control.

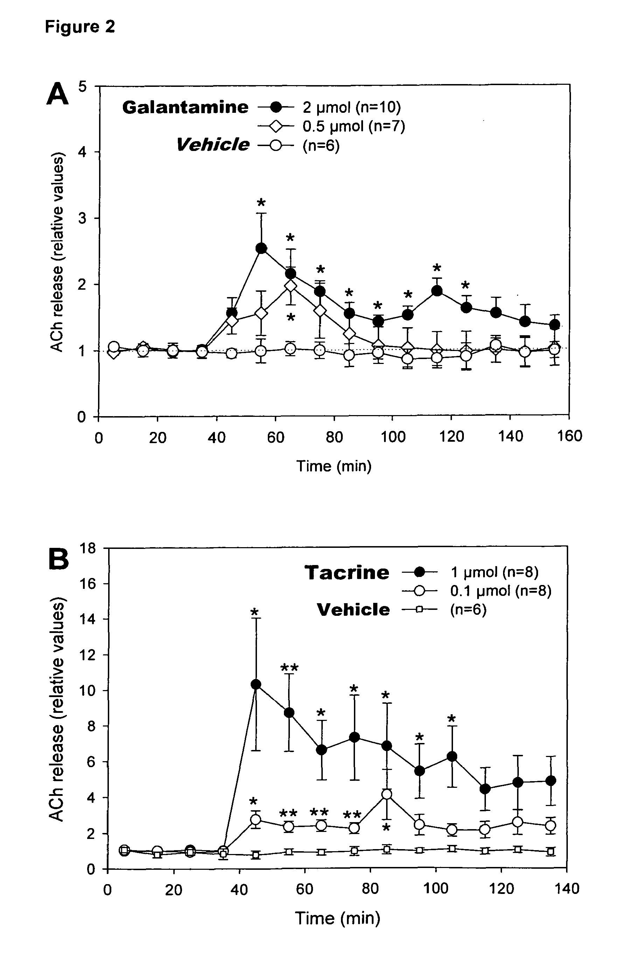

Fig. 2 shows the effects of galantamine (A; 2 and 0.5 µmol) and tacrine (B; 1 and 0.1 µmol) on extracellular ACh concentration in the nucleus accumbens. Arrows indicate time when i.c.v. injection (10 µl at 2 µl/min) of galantamine or tacrine solutions was started. Data are shown as relative values of basal release which is the mean of four pre-injection samples. Basal release is taken as 1.0 and indicated by a dotted line. **p* < 0.05, Friedman's analysis of variance followed by Dunn's multiple comparisons test using the pre-injection sample as control.

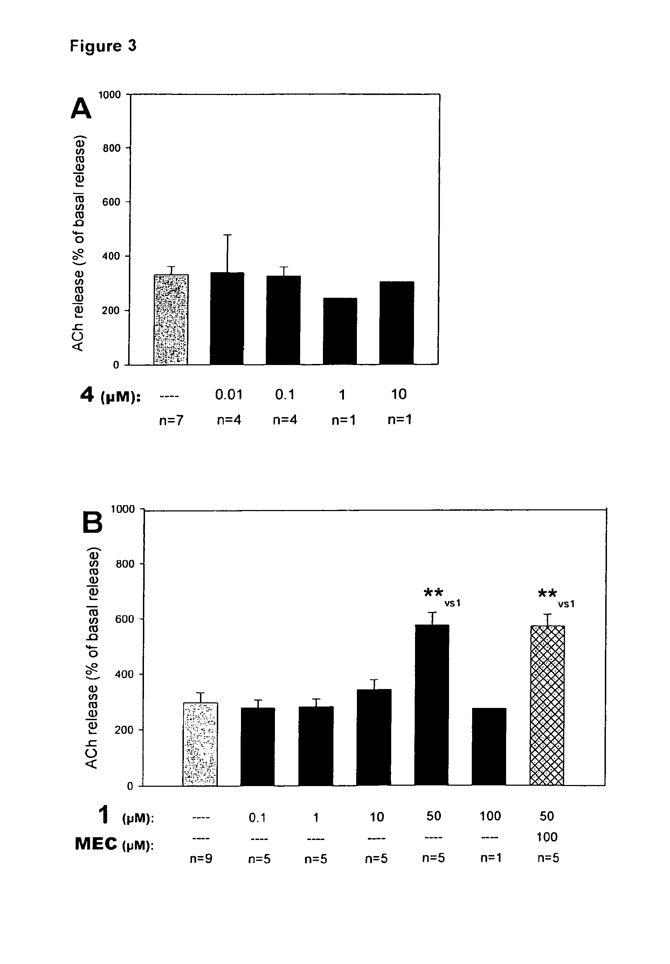

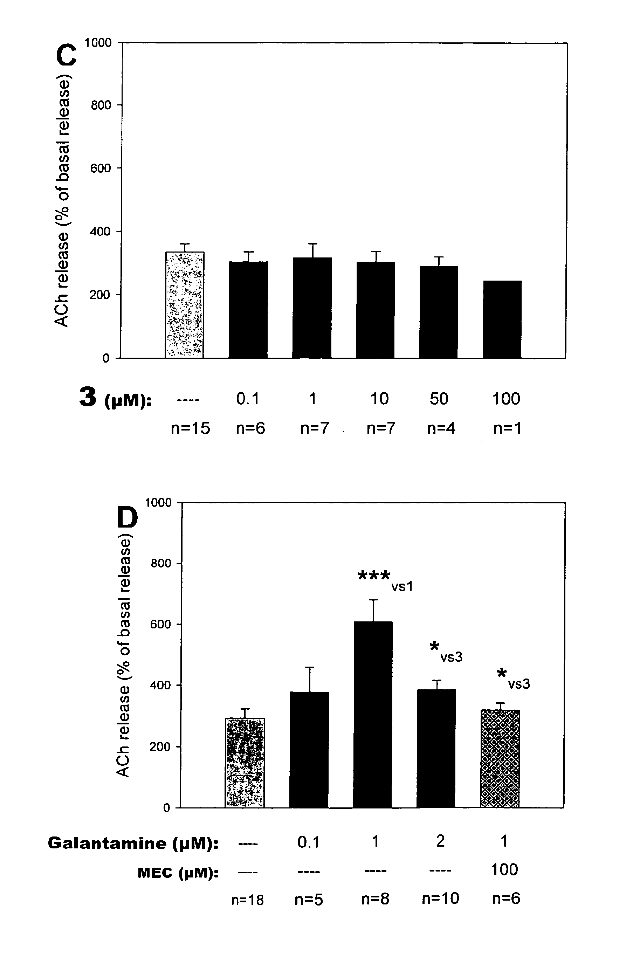

Fig. 3 shows the effect of the morphinan-6-one compounds and galantamine on 15 mM K+-induced release of ACh from frontal cortex synaptosomes. A: 4, B: 1, C: 3, D: galantamine. nAChRs were blocked with mecamylamine (MEC). Data are shown as means ± S.E.M. Analysis of variance ANOVA followed by Bonferroni test. **p*<0.05, **p*<0.01*,* p<0.001 compared to compound-free control group.

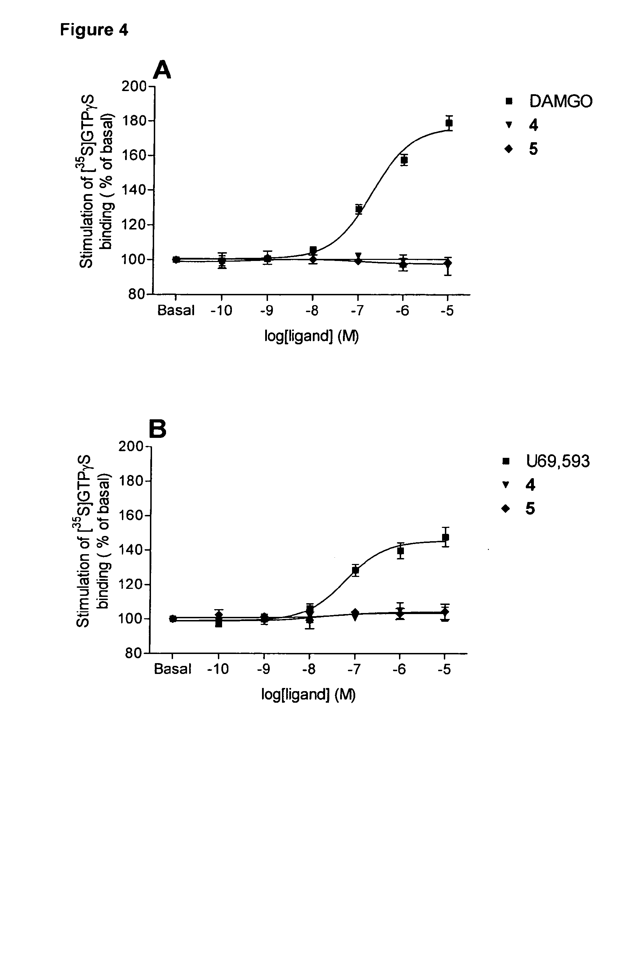

Fig. 4 shows the concentration-dependent effect of the compounds 4 and 5 on [³⁵S]GTP_{Y}S binding in comparison with the µ receptor agonist DAMGO in rat brain preparations (A) and in comparison with the _{K} opioid agonist U69,593 in guinea pig brain preparations (B). Data are shown as mean ± SEM of at least three independent experiments carried out in triplicate.

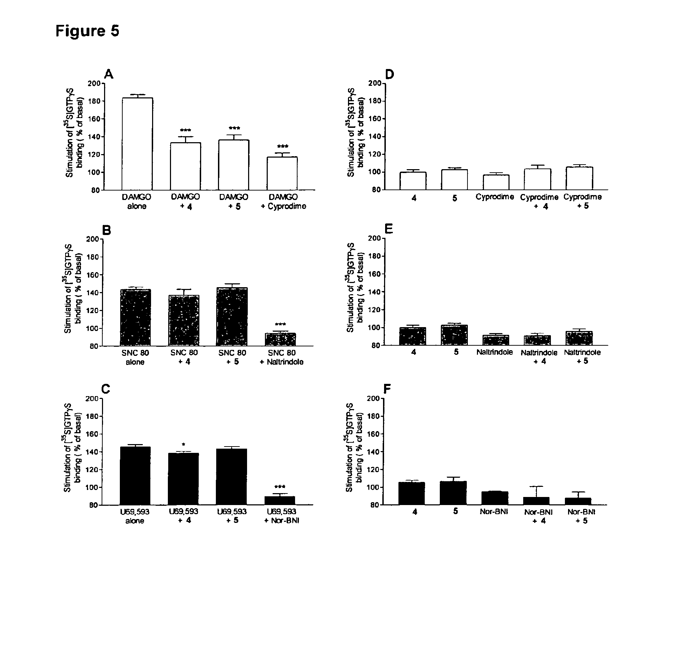

Fig. 5 shows the effect of compounds 4 and 5 on [³⁵S]GTPyS binding in the presence of selective opioid agonists (DAMGO, µ SNC 80, δ; U69,593, k) and selective opioid antagonists (cyprodime µ; naltrindole, δ; Nor-BNI k) . (A) DAMGO (10 µM) was incubated alone or in the presence of 4 (10 µM), 5 (10 µM) and cyprodime (10 µM) in rat brain membranes. (B) SNC 80 (10 µM) was incubated alone or in the presence of 4 (10 µM), 5 (10 µM) and naltrindole (10 µM) in rat brain membranes. (C) U69,593 (10 µM) was incubated alone or in the presence of 4 (10 µM), 5 (10 µM) and Nor-BNI (10 µM) in guinea pig brain membranes. (D) 4 (10 µM), 5 (10 µM) and cyprodime (10 µM) were incubated alone or in combination in rat brain membranes. (E) 4 (10 µM), 5 (10 µM) and naltrindole (10 µM) were incubated alone or in combination in rat brain membranes. (F) 4 (10 µM), 5 (10 µM) and nor-BNI (10 µM) were incubated alone or in combination in guinea pig brain membranes. Data are shown as mean ± SEM of at least three independent experiments carried out in triplicate. ****p*<0.001 vs. opioid agonist alone (A-C).

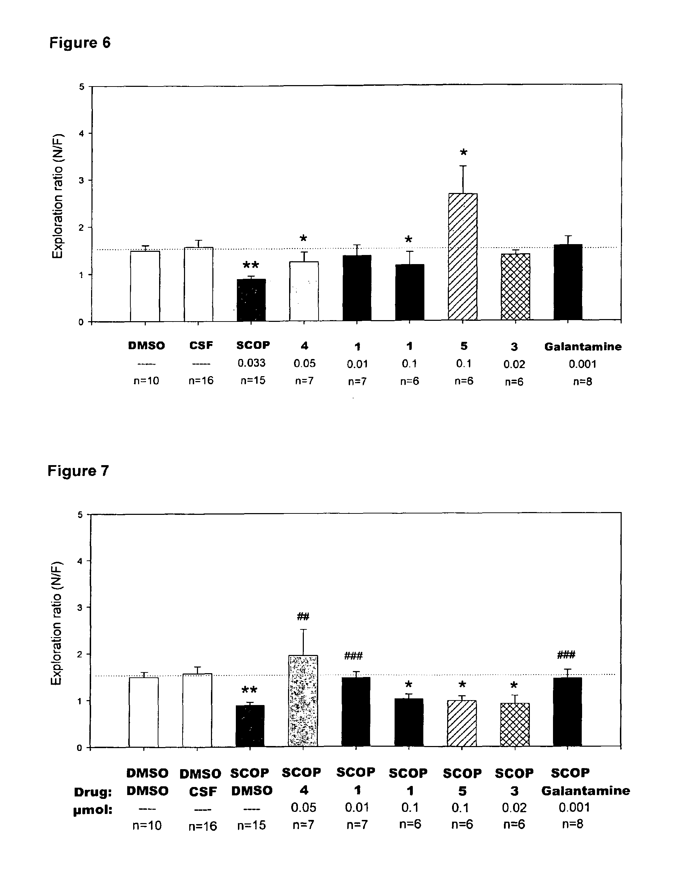

Fig. 6 shows the effect of scopolamine (SCOP), morphinan-6-one compounds 1, 3, 4, 5, and galantamine on object recognition. The drugs were injected i.c.v. in 2 µl of DMSO, except for 4 which was dissolved in 2 µl aCSF. Data are shown as means ±S.E.M. Analysis of variance ANOVA followed by multiple comparison Bonferroni test. ^{*} p<0.05, p<0.01 compared to the respective vehicle group.
Fig. 7 shows the effect of morphinan-6-one compounds 1, 3, 4, 5 and galantamine on scopolamine (SCOP)-induced amnesia. Dose of SCOP was 0.033 µmol. The drugs were injected i.c.v. in 2 µl of DMSO, except for 4 which was dissolved in 2 µl aCSF. Data are shown as means ±S.E.M. Analysis of variance ANOVA followed by multiple comparison Bonferroni test. * *p*<0.05, ***p*<0.01 compared to the respective vehicle group, ## p<0.01, ### p<0.001 compared to the scopolamine group.

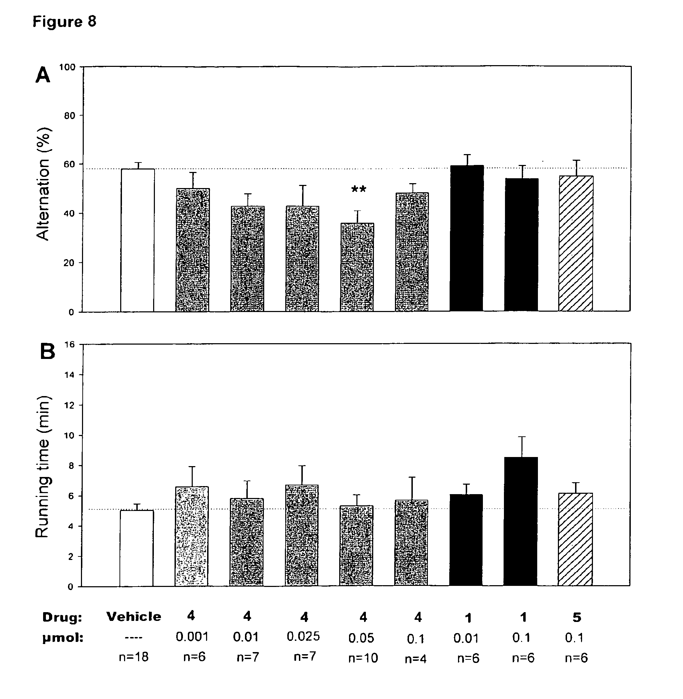

Fig. 8 shows the effect of morphinan-6-one compounds on alternation behaviour of mice in T-maze. A: spontaneous alternation (%), B: Running time (min). I.c.v. injection of 4 dissolved in 2 µl aCSF, 1 and 5 in 2 µl DMSO. Data of vehicles showed no difference and were pooled. Data are shown as means ± S.E.M. Analysis of variance ANOVA followed by Bonferroni test. ***p*<0.01 compared to vehicle group.

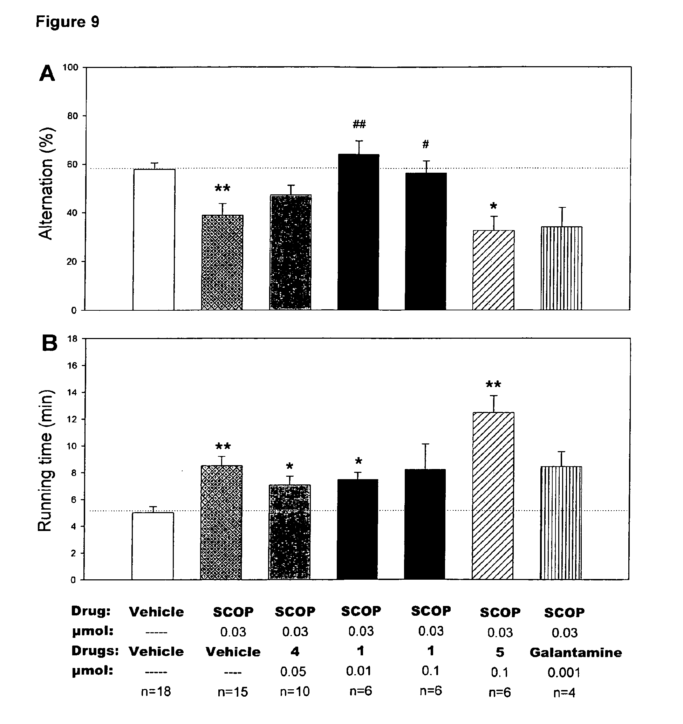

Fig. 9 shows the effect of morphinan-6-ones on scopolamine (SCOP)-impaired alternation behaviour of mice in T-maze. A: spontaneous alternation (%), B: Running time (min). I.c.v. injection of 4 dissolved in 2 µl aCSF, 1 and 5 in 2 µl DMSO. Data of vehicles showed no difference and were pooled. Data are shown as means ± S.E.M. Analysis of variance ANOVA followed by Bonferroni test. **p*<0.05, ***p*<0.01 compared to vehicle group, #*p*<0.05 and ##p<0.01 compared to scopolamine group.

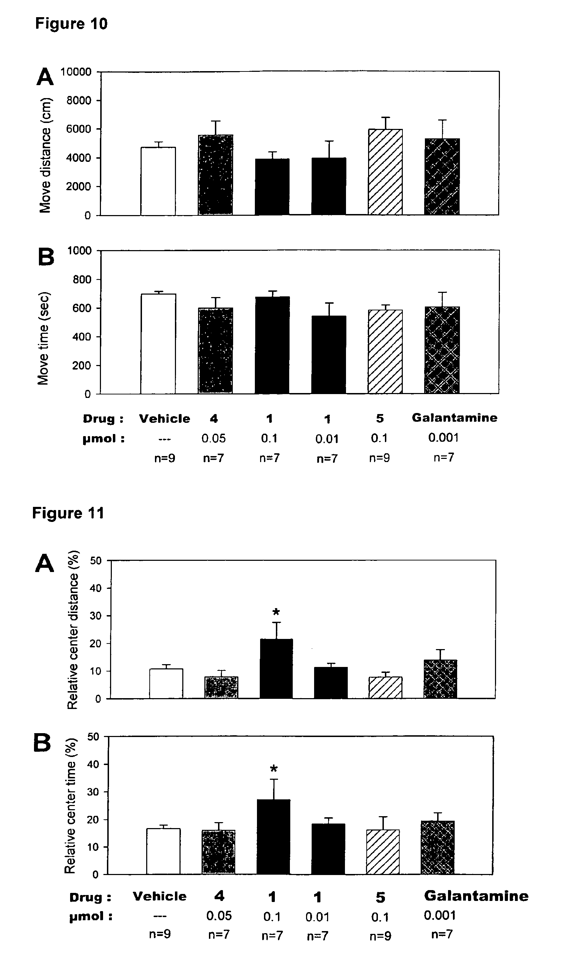

Fig. 10 shows the effect of 1, 4, 5 and galantamine on locomotor activity. A: move distance, B: move time. 4 was dissolved in 2 µl aCSF, 1, 5 and galantamine in 2 µl DMSO. Data of vehicles showed no difference and were pooled. Data are shown as means ± S.E.M. Analysis of variance ANOVA followed by Bonferroni test.
Fig. 11 shows the effect of 1, 4, 5 and galantamine on locomotor activity in center area. A: relative center distance, B: relative center time. 4 was dissolved in 2 µl aCSF, 1, 5 and galantamine in 2 µl DMSO. Data of vehicles showed no difference and were pooled. Data are shown as means ± S.E.M. Analysis of variance ANOVA followed by Bonferroni test. **p*<0.5 compared to vehicle group.

In table 1, preferred morphinan-6-one compounds according to the present invention are listed. The indicated numbering is kept throughout the examples.

**Table 1: Compound numbers, names and structure.**

| **Number** | **Name** | **Structure** |
|---|---|---|
| 1 | 1,4-dihydroxy-*N*-formylmorphinan-6-one | 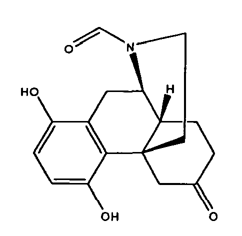 |
| 2 | 1,4-dione-/\/-formylmorphinan-6-one | 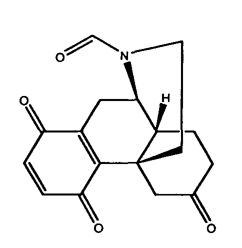 |
| 3 | 4,5-epoxy-*N*-formylmorphinan-6-one | 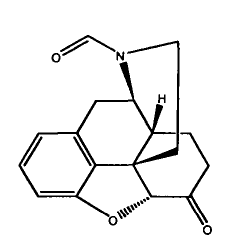 |
| 4 | 4-methoxy-*N*-allylmorphinan-6-one | 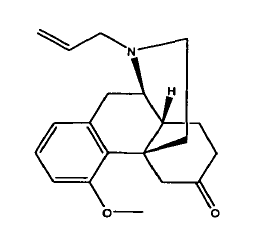 |
| 5 | 4,14-dimethoxy-/\/-allylmorphinan-6-one | 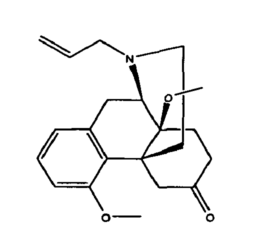 |

### Example 1

### Compounds according to the present invention possess cholinergic enhancing properties

### Release of brain Ach in vivo

### Methods

Push-pull technique: Sprague-Dawley rats were anaesthetized with urethane 1.2 g/kg, intraperitoneal. The head was fixed in a stereotaxic frame and a push-pull cannula (outer tubing: outer diameter 0.75 mm, inner diameter 0.41 mm; inner tubing: outer diameter 0.26 mm, inner diameter 0.13 mm) was mounted on a microdrive and stereotaxically inserted through a hole in the skull into the ventral striatum/nucleus accumbens (coordinates, mm from bregma: AP, +1.2; L, +2.5; V, 7.8). A second hole was drilled into the skull for the i.c.v. (into lateral cerebral ventricle) application of compounds (coordinates, mm from bregma: AP, -2.8; L, +1.5). The nucleus accumbens was superfused at 20 µl/min with artificial cerebrospinal fluid (aCSF), pH 7.2. The aCSF consisted of: 140 mmol/l NaCl; 3 mmol/l KCI; 1.2 mmol/l CaCl₂; 1 mmol/l MgC1₂; 1 mmol/l Na₂HPO₄; 0.3 mmol/l NaH₂PO₄; 3.0 mmol/l glucose; 1.0 µmol/l neostigmine. After 1 h of the cannula insertion the samples were collected into tubes kept at -20 °C in time periods of 20 min and then stored at -80 °C until ACh was determined.

Application of compounds: Compound 4 and tacrine were dissolved in 10 µL of aCSF, 1, 2, 3, 5, and galantamine were dissolved in 10 µl DMSO. Using a motorized microsyringe connected to a stereotaxic microdrive, the tip was introduced with an angle of 60° through the drug application-hole into the ipsilateral ventricle (coordinates mm from bregma: AP, -0.9; L, +1.5; V, -3.8) via a cannula (outer diameter 0.65; inner diameter 0.38). The drug solutions were injected at 2 µl/min. The control rats were injected with the respective vehicles (aCSF or DMSO).

Determination of ACh: ACh content in the samples was determined by injecting 100 µl into a HPLC system with a cation-exchanging analytical column (80 x 3 mm, Kromasil® 100-5 C18 loaded with laurylsulphate) and a post-column reactor (20 x 1 mm Kromasil® 100-10 NH₂ to which AChE and choline oxidase EC 1.1.3.17 were bound) followed by electrochemical detection at a UniJet platin electrode (3mm) at +500 mV with an amperometric detector (BAS LC-4, Bioanalytical Systems, West Lafayette, IN, USA). The mobile phase was 100 mM K₂HPO₄; 5 mM KCI; 1 mM tetramethylammonium hydroxide; 0.1 mM Na-EDTA and 0.5 ml/l Kathon^{R} CG, pH adjusted to 7.9 with H₃P0₄, pumped at a flow rate of 0.4 ml/min. The detection limit for ACh (signal to noise ratio = 3) was 10 fmol per sample. ACh was quantified with external standards that were injected at the beginning and the end of the analysis.

Statistical analysis: Statistical significance of compound effects on ACh release was calculated by Friedman's analysis of variance, followed by Dunn's post test using the mean release rate before the administration of compounds as controls. Data are shown as means ± SEM of relative values. The mean release rate before drug administration was taken as 1.0.

### Results

To investigate the procholinergic effect in the brain of the morphinan-6-one compounds according to the present invention, they were injected i.c.v. and the synaptic availability of ACh in the nucleus accumbens was monitored with the push-pull technique. Injection of 0.5 µmol of compound 4 induced a short, delayed increase of ACh in the superfusate (Fig. 1A, Table 2). At the dose of 0.5 µmol 2 caused a short increase in ACh concentration followed by a decrease (Fig. 1 B). The higher doses of 1 µmol and above of morphinan-6-ones 4 and 2 could not be tested because of toxicity. The dose of 0.25 µmol was ineffective (n=4, not shown). Compound 1 exerted a dose-dependent increase in extracellular brain ACh. A dose of 1 µmol enhanced the level of ACh to a peak value of 324±39 % and an AUC (area under the curve) of 1117±214 % of the basal value (Fig. 1C, Table 2). Compound 5 at the concentration of 0.5 µmol did not increase brain ACh (n=4, not shown) and 1 µmol of the drug enhanced ACh by a short peak increase which was very similar to that of 4 and 2 (Fig. 1 D). Due to the low number of experiments (n=4), the data of 5 did not reach statistical significance (Fig. 1 D). Compound 3 at the dose of 1 µmol increased ACh release while the dose of 2 µmol did not influence ACh up to 160 min followed by a decline thereafter (Fig. 1 E). The two AChE inhibitors galantamine and tacrine caused pronounced and dose-dependent increases in ACh release in the nucleus accumbens (Fig. 2A and B). The vehicles (aCSF for 4 and tacrine, and DMSO for the other compounds) did not influence the ACh concentration in the superfusate (DMSO: Fig. 2A, aCSF: Fig. 2B). The peak values and AUCs (0 to 120 min) of the compound-induced ACh release are shown in Table 2.

**Table 2: Peak values and AUCs of the compound-induced ACh release.**

| **Compound** | **Dose (µmol)** | **Increase in ACh release** (**Peak increase as % of basal output)** | **₀AUC₁₂₀ min (% increase x min) Mean ± SEM** |
|---|---|---|---|
| 1 | 0.5 | 65 ± 16* | 210 ± 31*** |
| | 1 | 224 ± 39** | 1017 ± 214*** |
| 2 | 0.5 | 57 ± 14* | 98 ± 27** |
| 3 | 1 | 123 ± 91* | 410 ± 227* |
| | 2 | 75 ± 73 | 160 ± 140 |
| 4 | 0.25 | inactive | inactive |
| | 0.5 | 43 ± 19* | 85 ± 26** |
| 5 | 1 | 51 ± 43 | 158 ± 111 |
| **Galan-tamine** | 0.5 | 118 ± 29* | 642 ± 85** |
| | 1 | 101 ± 42* | 1857 ± 173*** |
| **Tacrine** | 0.1 | 197 ± 45* | 1729 ± 238*** |
| | 1 | 913 ± 371* | 6143 ± 870*** |

| | | | |
|---|---|---|---|
| * p<0.05, **p<0.01, ***p<0.001 from basal release. | | | |

### ACh release from synaptosomes

### Methods

Preparation of synaptosomes: synaptosomes were prepared from brain frontal cortex of male Sprague Dawley rats (4 months old). The frontal cortex was rapidly dissected on ice and homogenized in 40 volumes of 0.32 M sucrose buffered at pH 7.4 with 0.01 M sodium phosphate. The homogenate was centrifuged at 1,000 g for 5 min. The crude synaptosomal pellet was obtained by centrifugation of the supernatant at 15,000 g for 20 min and was resuspended in 5 ml basal medium (125 mM Nacl, 3 mM KCI, 1.2 mM MgS0₄, 1.2 mM CaCl₂, 1.0 mM NaH₂PO₄, 22 mM NaHCO₃ and 10 mM glucose) which had been gassed with 95% O₂ - 5% CO₂ at 37 °C, pH 7.2 - 7.4.

Superfusion protocol: The synaptosomal suspension was incubated with [³H]-choline (0.03 µM, 17.5x10⁶ Bq/g) for 15 min at 37 °C. Then, the suspension was diluted (1:8) and aliquots were applied on filters in a continuous superfusion system with parallel chambers maintained at 37 °C. The media used for superfusion were basal medium and 15 mM K⁺-containing stimulation medium. Both media were supplemented with ACh (1 µM) to facilitate the effectiveness of an allosteric potentiating ligand at the nicotine receptor and with atropine (0.1 µM) to block M2 presynaptic receptors. Synaptosomes were superfused with basal medium for 30 min to reach a constant outflow of [³H]-ACh. Under these experimental conditions, the radioactivity released consisted largely of newly synthesized [³H]-ACh. The K⁺-stimulated release of [³H]-ACh was determined in 2 subsequently collected fractions: 6 min basal release (superfusion with basal medium) and 6 min K⁺-evoked release (superfusion for 1.5 min with 15 mM K⁺-containing medium followed by 3.5 min basal medium). The test compounds galantamine, 4 and 2 were present in the superfusion medium concomitantly with 15 mM K⁺ (for 1.5 min). Basal release of [³H]-ACh (continuous superfusion with basal medium) was determined by collection of a 6 min sample followed by four subsequent 2 min samples. Aliquots of samples were mixed with scintillation liquid and the radioactivity quantified with a scintillation counter. The radioactivity bound to the filters was also measured.

Statistical analysis: The amount of [³H]-ACh released in each sample was calculated as fraction of total synaptosomal tritium at the start of the respective collection period (tritium in the sample / sum of tritium in the respective fractions and in the synaptosomes on the filter). K⁺-evoked release was expressed as percentage of basal release rate. Each n=1 represents the mean of an experiment with one synaptosomal preparation in at least 3 superfusion chambers. Statistical significance of group variances was calculated by one way ANOVA followed by Bonferroni's test.

### Results

The control release of ACh from synaptosomes was evoked by superfusion with 15 mM K⁺-containing medium and amounted to about 300 to 350 % of basal, non-stimulated release (Fig. 3). This effect is half-maximal and is susceptible to modulation by activation or inhibition of presynaptic receptors which are present on the synaptosomes. Compound 2 and 5 were not tested because the required quantity was not available. Compound 4 (0.01 to 10 µM) and 3 (0.1 to 100 µM) did not influence ACh release in frontal-cortex synaptosomes (Fig. 3A, C). 1 enhanced the stimulation-evoked ACh release with the maximal effect at 50 µM. Lower (0.1 and 1 µM) and higher concentrations (100 µM) of 1 were ineffective. The nAChR (nicotinic acetylcholine receptor) antagonist mecamylamine did not influence the 50 µM of compound 1 induced release suggesting that nAChRs are not involved in this effect (Fig. 3B). The positive control galantamine enhanced the release of ACh showing a bell shaped concentration-effect curve with the maximal effective concentration of 1 µM. This effect was blocked by mecamylamine as expected from a substance characterized as nAChR allosteric agonist (Fig. 3D).

### Spectrophotometric assay for AChE inhibitory activity

### Method

The AChE inhibitory activity of the morphinan-6-one compounds of the present invention was determined using a modified Ellman's method with electric eel AChE (EC 3.1.1.7), acetylthiocholiniodide, and 5,5'-dithiobis-(2-nitrobenzoic acid) (Sigma-Aldrich, Germany), Galantamine.HBr (Tocris; Cookson Ltd, Avonmouth UK Bristol) served as the positive control in our assay (IC₅₀ of 3.2 ± 1.0 µM; IC₅₀ is the concentration necessary to produce 50% inhibition) using a 96-well microplate assay. The percentage of the enzyme inhibition was calculated by determining the rate in presence of inhibitor and the vehicle (containing 1% DMSO) compared to the rate in the control sample (n=4) and analyzed with Student's *t-*test.

### Results

All tested morphinan-6-one compounds of the present invention showed to be effective inhibitors of AChE activity as shown in Table 3.

### Example 2

### Compounds according to the present invention do not display opioid receptor agonism or sigma receptor binding

### Opioid receptor binding assay

### Method

Membrane fractions were prepared from Sprague-Dawley rat or guinea pig brains. Briefly, brains without cerebella were homogenised on ice in 5 volumes of ice-cold 50 mM Tris-HCI buffer (pH 7.4) and diluted in 30 volumes of the same buffer. After centrifugation at 40,000 x g for 20 min at 4°C, the pellets were resuspended in 30 volumes of Tris-HCI buffer and incubated at 37 °C for 30 min (rat brain) or for 10 min (guinea pig brain). The centrifugation step was repeated and the final pellets were resuspended in 5 volumes of 50 mM Tris-HCI buffer (pH 7.4) containing 0.32 M sucrose to give a protein concentration of 4 - 6 mg/ml. Protein concentration was determined by the method of Bradford using bovine serum albumin as standard.

Binding experiments were performed in 50 mM Tris-HCI buffer (pH 7.4) in a final volume of 1 ml containing 0.3 - 0.5 mg protein and different concentrations of the compounds. Rat brain membranes were incubated either with [³H]DAMGO (45 min, 35 °C) or [³H][IIe^{5,6}]deltorphin II (45 min, 35 °C). Guinea pig brain membranes were incubated with [³H]U69,593 (30 min, 30 °C). Non-specific binding was determined in the presence of 10 µM unlabeled naloxone. Reactions were terminated by rapid filtration through either Whatman glass fibre filters GF/B pretreated with 0.1% polyethylenimine (1³H]U69,593) or GF/C ([³H]DAMGO and [³H][IIe^{5,6}]deltorphin II) using a Brandel M24R Cell Harvester, followed by three washings with 5 ml of ice-cold 50 mM Tris-HCI buffer (pH 7.4). Inhibition constant (Kᵢ) values were calculated from competition binding curves using GraphPad Prism (San Diego, CA, USA) program. The values are expressed as the mean ± S.E.M of 2-4 independent experiments, each performed in duplicate.

### Results

The morphinan-6-one compounds of the present invention were evaluated in in vitro receptor binding assays for their interaction abilities with µ, δ and k opioid receptors. The binding affinities at µ and δ opioid receptors were determined by inhibition of binding of [³H][D-Ala², Me-Phe ⁴, Gly-ol⁵]enkephalin (hereinafter [³H]DAMGO) and [³H][lle^{5,6}]deltorphin II to rat brain membranes. The affinities of the compounds at k opioid receptors were determined by displacement of [³H]U69,593 using guinea pig brain membranes. The µ, δ and k opioid receptor binding affinities are expressed as inhibition constant (Kᵢ) and are summarized in Table 3.

.

**Table 3: AChE inhibitory activity and opioid receptor binding affinities of compounds 1 to 5.**

| **Compound** | **AChE inhibitory activity** I**C₅₀ (µM)** | **Opioid binding affinities Kᵢ (nM)** | | |
|---|---|---|---|---|
| | | **µ receptor** | **δ receptor** | **κ receptor** |
| **1** | 105 | >10,000 | >10,000 | >10,000 |
| **2** | 12.7 | >10,000 | >10,000 | >10,000 |
| **3** | 60.3 | >10,000 | >10,000 | >10,000 |
| **4** | 1.0 | 20.2 | 316 | 33.1 |
| **5** | 81.9 | 67.2 | 1253 | 328 |

The N-formylmorphinan-6-ones 1, 2, and 3 showed no specific binding to any of the three opioid receptor types (Kᵢ > 10 µM). Whereas the morphinan-6-one compounds 4 and 5 showed both some µ opioid receptor affinity. The nature of this affinity (agonistic or antagonistic) was further investigated in a functional assay (see below). [³⁵S]GTPyS functional assay

### Method

Membrane fractions were prepared from Sprague-Dawley rat or guinea pig brains as described above. To determine binding of [³⁵S]GTPyS, rat or guinea pig brain membranes (10 µg of protein) were incubated for 60 min at 30 °C in Tris-EGTA buffer (50 mM Tris-HCl buffer, 3 mM MgCl₂, 1 mM EGTA, 100 nM NaCl, pH 7.4) containing 30 µM GDP, 0.05 nM [³⁵S]GTPyS and appropriate concentrations of the respective compounds according to the present invention in a final volume of 1 ml. The reaction was terminated by vacuum filtration through Whatman GF/B glass fibre filters. Filters were washed three times with ice-cold 50 mM Tris-HCI buffer (pH 7.4) and the bound radioactivity was measured by liquid scintillation counting (Beckman Coulter™ LS6500). Basal activity was determined by subtracting the non-specific binding (measured in the presence of 100 µM unlabelled GTPyS) from the total binding (measured in the absence of the compound).

Stimulation produced by the compound is given as percentage of the basal activity (given as 100%). Functional data, EC₅₀ (nM; half maximal effective concentration) and maximal stimulation Eₘₐₓ (% stimulation over basal level; maximal effective concentration), were calculated by using nonlinear regression analysis and sigmoidal curve fitting GraphPad Prism (San Diego, CA, USA) program. The values are expressed as the mean ± S.E.M of at least 3 independent experiments, each performed in triplicate. Statistical analysis was carried out using SPSS software (SPSS Inc., Chicago, lL, USA) and the Student *T*-test was used to compare different assay conditions. A *ρ* value < 0.05 was considered statistically significant.

### Results

The [³⁵S]GTPyS binding assay was used to assess in vitro the functional activity at opioid receptors of the selected morphinan-6-ones. Only compounds 4 and 5 which display specific binding at opioid receptors with moderate selectivity for the µ type (Table 3), were functionally investigated. As shown in Fig. 4, no increase in [³⁵ S]GTP_{Y}S binding was found in the presence of morphinans 4 and 5 in rat or guinea pig brain membranes, thus indicating that they have antagonist properties. In contrast, concentration-dependent stimulation of [³⁵ S]GTP_{Y}S binding was produced by the prototype opioid agonists with selectivity for the µ opioid receptor, DAMGO (Fig. 4A; EC₅₀ of 297 ± 40 nM and Eₘₐₓ of 179 ± 5%) or the δ receptor, SNC 80 (data not shown; EC₅₀ of 71.2 ± 9.7 nM and Eₘₐₓ of 137 ± 4%) assessed in rat brain membranes, or for the _{K} receptor, U69,593 (Fig. 4B) (EC₅₀ of 60.7 ± 4.4 nM and Eₘₐₓ of 145 ± 5%) in guinea pig brain preparations.

The opioid receptor type involved in the antagonist activity of compounds 4 and 5 was investigated by measuring their ability to inhibit [³⁵ S]GTP_{Y}S binding stimulated by selective µ, δ and k opioid agonists, and comparing the antagonism to the effects of µ, δ and k selective opioid antagonists. As shown in Fig. 5A, the increase of [³⁵S]GTPγS binding by the µ opioid agonist DAMGO (10 µM) was significantly decreased by both compounds 4 (10 µM) and 5 (10 µM), an effect which was similar to that produced by the µ receptor selective antagonist cyprodime (10 µM). Compound 4 (10 µM) and 5 (10 µM) did not cause any significant changes in the increased [³⁵S]GTPyS binding by the µ opioid agonist SNC 80 (10 µM), while naltrindole (10 µM), a selective δ opioid antagonist, markedly decreased SNC 80 stimulatory effect (Fig. 5B). While no significant effect was produced by compound 5 (10 µM) on stimulated [³⁵S]GTPyS binding by the k opioid agonist U69,593 (10 µM), a slight, but significant decrease was produced by 4 (10 µM). The prototype _{K} selective opioid antagonist Nor-BNI (10 µM) was highly effective in inhibiting the U69,593-stimulated [³⁵S]GTPyS binding (Fig. 5C). Furthermore, co-incubation of compounds 4 and 5 with the selective opioid antagonists, cyprodime (µ) (Fig. 5D), naltrindole (δ) (Fig. 5E) or Nor-BNI (k) (Fig. 5F) did not produce any significant increase in [³⁵S]GTPyS binding when compared to the antagonist alone, suggesting the absence of agonist activity.

### Sigma receptor binding assay

### Method

The sigma receptor binding assay was performed with guinea pig brain membranes prepared as described above. Membranes (0.3 - 0.5 µg) were incubated in 50 mM Tris-HCI buffer (pH 8) with [³H](+)-pentazocine or [³H]DTG (radioligand concentration of 1 - 2 nM) in a final volume of 1 ml and at least 10 different concentrations of the respective compounds according to the present invention. Non-specific binding was determined in the presence of 10 µM haloperidol. Incubations were carried out for 120 min at 25 °C and were terminated by rapid filtration through Whatman glass fibre filters GF/B pretreated with 0.1 % polyethylenimine for 30 min at room temperature using a Brandel M24R Cell Harvester. Filters were washed three times with 5 ml of ice-cold 10 mM Tris-HCI buffer (pH 8). The bound radioactivity was measured by liquid scintillation counting (Beckman Coulter™ LS6500). Binding data (IC₅₀, concentration necessary to produce 50% inhibition) were analysed using the non-linear least-square sigmoidal curve fitting by GraphPad Prism program (San Diego, CA, USA). The values are expressed as the mean ± S.E.M of 2 - 4 independent experiments, each performed in duplicate.

### Results

As indicated in Table 4, none of the five tested morphinan-6-one compounds (1 - 5) showed any specific binding to the σ receptors labelled by [³H](+)-pentazocine (labels sites with σ1 profile) or [³H]DTG (labels both σ1 and σ2 sites) using competition binding assays in guinea pig brain membranes. In contrast, all the reference σ receptor ligands displayed specific binding (Table 4). These results showed that none of the morphinan-6-one compounds bind to sigma receptors. Hence, these receptors do not play any role in their effects on ACh and mouse behaviour.

**Table 4: Sigma receptor binding affinities of compounds 1 to 5 and reference σ receptor ligands.**

| **Ligand** | **IC₅₀ ± SEM (nM)** | |
|---|---|---|
| | **[³H](+)pentazocine (σ1)** | **[³H]DTG (σ1** + **σ2)** |
| **(±)Pentazocine** | 15.6 ±1.4 | 54.6 ± 5.3 |
| **Haloperidol** | 3.20 ± 0.1 | 7.62 ± 1.4 |
| **DTG** | 130 ± 20 | 126 ± 5 |
| **(+)-SKF-10,047** | 203 ± 7 | 291 ± 14 |
| **(+)-3-PPP** | 68.2 ± 4.8 | n.d. |
| **1** | > 10,000^{a} | >10,000^{a} |
| **2** | >10,000^{a} | >10.000^{a} |
| **3** | >10,000^{a} | >10,000^{a} |
| **4** | >10.000^{a} | >10,000^{a} |
| **5** | >10,000^{a} | >10,000^{a} |

| | | |
|---|---|---|
| n.d., not determined.^{a} tested up to 10 µM. | | |

### Example 3

### Compounds according to the present invention can abolish induced cholinergic memory impairment

### General procedures

Animals: C57BL/6N female mice (3 months old) were housed in groups of six in cages (40 (L) x 25 (W) x 15 (H) cm) placed in a room at a constant temperature (22±1 °C), 12 h-light / 12 h-dark cycle (07:00-19:00 h), with food and water ad libitum. Experiments were conducted between 08:00 and 16:00h.

Surgery: Mice were anaesthetized with pentobarbital sodium (60 mg/kg, intraperitoneal) and placed in a stereotaxic frame. A guide cannula (gauge 25, outer diamenter 0.52 mm, inner diameter 0.26 mm and 7 mm long) was stereotaxically implanted through a hole on the skull into the brain area 0.5 mm higher than the lateral cerebral ventricle (-0.1 mm posterior, 0.8 mm lateral to bregma, -2.2 mm ventral to the dura). Guide cannula was secured to the skull using two small screws and dental cement. A stainless steel obturator was placed inside each cannula to maintain patency. Each animal recovered for 5 days prior to experimentation.

Experimental design and drug treatment: Animals were randomly divided into experimental groups according to the treatments. Each mouse was used only once. Drugs were dissolved in 1 or 2 µl of the respective vehicles (scopolamine HBr in 1 µl DMSO, galantamine, 1, 5 and 3 in 2 µl DMSO, 4 in 2 µl aCSF). All drugs and vehicles were injected 15 min before the test (T-maze and open field) or before the training trial, T1, (object recognition test) through the 7.5 mm long injector connected with inner diameter 0.28 mm polyethylene tubing to 10 µl Hamilton syringe. The injector was left in place for an additional 1 min to allow diffusion of the drug away from the injector tip. Thereafter it was replaced by the obturator.

### Object recognition task experiments

### Method

Apparatus: The test apparatus consisted of a box made of Plexiglas (40 (L) x 25 (W) x 15 (H) cm), which was illuminated by a lamp above the box and surmounted by a video camera connected to a monitor and a video recorder. In the different parts of the apparatus, the light intensity was equal at 100 lux. The objects to be discriminated were made of plastic or metal, and in the different shapes: cubes or cylinders, 1.5 cm high in white or green colour. These objects had no genuine significance for mice and had never been associated with reinforcement.

Procedure: One day before testing, the animals were allowed to explore the apparatus for 10 min (without objects), while on the testing day a session of two 10 min trials was given. During the training trial (T1), two identical objects were placed on opposite sides 8 and 6 cm from the short and long walls, respectively. A mouse was placed in the middle of apparatus and was left to explore the two identical objects for 10 min. After T1, the mouse was put back in its home cage and a 3 h inter-trial interval was given. Subsequently, the testing trial (choice trial; T2), was performed for 10 min. During T2, a novel object (N) replaced one of the samples presented in T1, hence, the mouse was now exposed to two different objects: the familiar (F) and the novel one (N). All combinations and locations of objects were used in a balanced manner to reduce potential biases due to preferences for particular locations or objects. To avoid the presence of olfactory trails, the apparatus and the objects were thoroughly cleaned with water and dried after each trial.

Exploration was defined as follows: directing the nose toward the object at a distance of less than 0.5 cm and/or touching the object with the forepaw or nose. Turning around or sitting on the object was not considered as exploratory behaviour.

The times spent by mice in exploring each object during the experiment were recorded and the video tapes were analyzed by using a stopwatch. From this measure a series of variables was then calculated: the total time spent with exploring the two identical objects in T1 and that spent with exploring the two different objects, familiar and novel in T2. The discrimination between the familiar and the novel object during T2 was measured by comparing the time spent with exploring the familiar sample with that spent with exploring the new object. As this time may be biased by differences in overall levels of exploration, a discrimination index (D) was then calculated; D=N-F/N+F. D is the discrimination index and represents the difference in exploration time expressed as a proportion of the total time spent with exploring the two objects in T2. The object exploration time ratio (E) was calculated as object exploration time with a novel object during testing divided by object exploration time with a familiar object during testing; E=N/F. Such ratio is considered to be the main measure of retention in this task.

### Results

First, the mice were exposed to a pair of identical objects and thereby learned to recognize this type of object. After the interval of 3 hours, a novel object together with one of the familiar ones was presented. Recognition of the familiar object is revealed by a lower exploration time compared to the exploration time for the novel one. The data shown are the ratio of exploration times novel/familiar object. Normal mice without implanted i.c.v. cannula showed an exploration ratio of 1.479 ± 0.107 (mean ± S.E.M., n=12; not shown). Implantation of i.c.v. cannula and injection of the vehicles DMSO or aCSF did not influence object recognition (DMSO: 1.409 ± 0.113, n=10; CSF: 1.578 ± 0.107; n= 16; Fig. 6). Fig. 6 shows the effect of the compounds on normal mice, without previous pretreatment. Scopolamine (0.03 µmol) successfully induced amnesia since the mice did not discriminate between novel and familiar object as shown by an exploration ratio of close to one. Compound 4 (0.05 µmol) induced a small impairment of memory. Compound 1 at the dose of 0.01 µmol did not influence memory while 0.1 µmol caused a significant impairment. 5 (0.1 µmol) increased the exploration ratio suggesting an improvement of object recognition. 3 (0.02 µmol) and galantamine (0.001 µmol) did not influence the object recognition (Fig. 6). The effect of the tested morphinan-6-ones and galantamine on the amnesia induced by scopolamine is shown in Fig. 7. Compounds 4 (0.05 µmol), 1 (0.01 µmol) and galantamine (0.001 µmol) abolished the scopolamine-induced impairment of object recognition, as shown by exploration ratios which were not different from control values. 5 and 3 were ineffective in the scopolamine-pretreated animals (Fig. 7).

### T-maze continuous alternation experiments

### Method

Apparatus: The T-maze was made of grey perspex with three guillotine doors which could be operated by the experimenter. The arms were 12 cm wide, the two arms aside from the left and right guillotine door (goal arms) were 31 cm long. The length of the central arm (start arm, stem of the T) was 76 cm including a compartment of 24 cm in front of the entrance door.

Procedure: Testing of a mouse consisted of one single session, which started with 1 forced-choice trial, followed by 14 free-choice trials. In the first trial, the forced-choice trial, either the left or right goal arm was blocked by lowering the guillotine door. After the mouse was released from the start arm, it explored the maze, eventually entered the open goal arm, and returned to the start position. There, the animal was confined for 5 s by lowering the guillotine door of the start arm. During 14 free-choice trials, the mouse could choose freely between the left and right goal arm. After opening the guillotine door of the start arm the animal was free to choose between both goal arms (all guillotine doors open). As soon as the mouse entered one goal arm, the other goal arm was closed. The mouse eventually returned to the start arm, and the next free-choice trial started after a 5 s confinement in the start arm. A session was terminated and the animal was removed from the maze as soon as 14 free-choice trials had been performed or 30 min had elapsed, whatever event occurred first. During the session, the animals were never handled by the experimenter. In order to isolate the cognitive component, care was taken to diminish the odour cues present by cleaning and drying the maze with paper towels after each test, as these cues are likely to influence the alternation rate. The alternation score for each mouse was calculated as the ratio of the actual number of alternation to the possible number (defined as the total number of arm entries minus two) multiplied by 100.

### Results

After one forced-choice trial in which one T-arm of the maze was closed, mice were given the opportunity to carry out 14 free-choice trials with both T-arms open. Normal mice injected with vehicle showed a spontaneous alternation rate of 61.04 ± 3.01 (mean ± SEM, n=18). I.c.v. injection of the morphinan-6-one 4 at the dose of 0.05 µmol decreased spontaneous alternation rate. Lower (0.001; 0.01 and 0.025 µmol) or higher doses (0.1 µmol) of 4 did not exert a significant effect on spontaneous alternation. These data might suggest a negative bell-shaped dose-effect curve of 4 on alternation rate (Fig. 8A). 1 (0.01 and 0.1 µmol) and 5 (0.1 µmol) did not influence alternation (Fig. 8A). These three morphinan-6-ones did not influence the time it took for the animals to finish the test (running time) (Fig. 8B). The anticholinergic compound scopolamine induced a dose-dependent reduction in spontaneous alternation and a strong increase in running time with a bell-shaped curve (not shown). The dose of 0.033 µmol of scopolamine was the lowest dose which induced a profound decrease in alternation and only a moderate increase in running time. This dose was used as pretreatment for testing the effect of the morphinan-6-ones of the present invention on scopolamine-impaired alternation.

Fig 9 shows the effect of the morphinan-6-ones on scopolamine-treated mice. The effect of vehicle and scopolamine alone (0.033 µmol) is also shown for comparison. 4 at the dose of 0.05 µmol ameliorated the profound scopolamine-induced reduction in alternation (Fig. 9A) while 0.01 µmol of the compound was ineffective (not shown). 1 (0.01 and 0.1 µmol) abolished the cholinergic impairment of alternation. Mice pretreated with scopolamine and injected with 1 showed an alternation rate that was not different from vehicle-treated controls (Fig. 9A). 1 did not influence the scopolamine-increased running time which remained prolonged (Fig. 9B). 5 (0.1 µmol) had no effect on scopolamine-impaired alternation but it further increased the scopolamine-enhanced running time (Fig. 9A, B). We observed that galantamine at the doses 0.005; 0.01 and 0.05 µmol impaired alternation performance of normal, untreated mice. It caused a large extension of the running time which prevented the accomplishment of the task (not shown). Mice treated with 0.001 µmol galantamine finished the task and alternated at rates not different from control animals (not shown). However this dose failed to ameliorate the reduced alternation of scopolamine-treated animals (Fig. 9B).

### Open field experiments

### Method

The open field consisted of a transparent plastic box with white floor (41 x 41 x 41 cm) equipped with an automated activity monitoring system (True Scan, Coulbourn Instruments, Allentown, Pennsylvania, USA). The illumination at floor level was 150 lux. Mice were individually placed into the centre of the open field and their behaviours were tracked with the activity monitoring system. The time of locomotor activity, the overall distance travelled by the mice and the fraction of locomotor activity in the center area (center distance and center time) were monitored for 10 min. The arena was thoroughly cleaned with wet paper towels and dried at the end of each test.

Histology: At the end of the experiment the rats and mice were killed with an overdose of urethane, a solution of cresyl violet was injected through the push-pull or i.c.v. cannulas. The brains were removed and stored in buffered formaldehyde solution (7 %). Serial coronal-sections were cut at 50 µm intervals. Localisations of the push-pull cannula and i.c.v. microinjection were verified on serial sections by the trace of the cannula and staining of the ventricle. Experiments with unsatisfactory localisation were discarded.

Statistical analysis: All data from behavioural studies are shown as mean ± SEM. Statistical analysis was performed using the analysis of variance (ANOVA), followed by post hoc multiple comparisons Tukey-Kramer test, except comparison of alternation rates with the theoretical value of chance (defined as 50%) which was evaluated by Wilcoxon signed rank test. All statistical analyses were carried out by using the Graphpad Prism Program, version 4.0, Graphpad software, Inc.

### Results

Normal mice injected i.c.v. with the vehicle showed a move distance of 4731 ± 389 cm and a move time of 697 ± 19 sec (mean values ± SEM, n=9). Injection of 4 (0.1 µmol), 1 (0.01 and 0.1 µmol), 5 (0.1 . µmol) and galantamine did not influence these parameters of general locomotion in the open field (Fig. 10A, B). The fraction of locomotor activity of vehicle treated mice in the center area was 10.7 ± 1.5 % of move distance and 16.7 ± 1.7 % of move time. Compound 1 at the dose of 0.1 µmol increased the relative center distance and move time significantly while the dose of 0.01 µmol had no effect on these parameters. 4, 5 and galantamine did not influence the fraction of locomotor activity in the central zone (Fig 11).

## Claims

1. A morphinan-6-one compound for the treatment or prevention of neurodegenerative diseases.

2. The morphinan-6-one compound of claim 1, which is a N-formylmorphinan-6-one.

3. The morphinan-6-one compound of claims 1 or 2, wherein the neurodegenerative disease is selected from the group consisting of Alzheimer's disease, Mild Cognitive Impairment, amyloidosis, Parkinson's disease, multiple sclerosis, amyotrophic lateral sclerosis, Huntington's disease, Lewy body dementia, Pick's disease, bovine spongiform encephalitis and Creutzfeldt-Jakob disease.

4. The *N*-formylmorphinan-6-one compound of claim 2 or 3, wherein the compound is 1,4-dihydroxy-N-formylmorphinan-6-one.

5. The N-formylmorphinan-6-one compound of claim 2 or 3, wherein the compound is 1,4-dione-N-formylmorphinan-6-one.

6. The N-formylmorphinan-6-one compound of claim 2 or 3, wherein the compound is 4,5-epoxy-*N*-formylmorphinan-6-one.

7. The morphinan-6-one compound of claim 1 or 3, which is 4-methoxy-N-allylmorphinan-6-one.

8. The morphinan-6-one compound of claim 1 or 3, which is 4,14-dimethoxy-N-allylmorphinan-6-one.

9. The use of a morphinan-6-one compound for the preparation of a pharmaceutical composition for the treatment or prevention of neurodegenerative diseases.

10. The use according to claim 9, wherein the compound is 1,4-dihydroxy-N-formylmorphinan-6-one.

11. The use according to claim 9, wherein the compound is 1,4-dione-N-formylmorphinan-6-one.

12. The use according to claim 9, wherein the compound is 4,5-epoxy-*N*-formylmorphinan-6-one.

13. A pharmaceutical composition comprising at least one morphinan-6-one compound and at least one pharmaceutically acceptable excipient for the treatment or prevention of neurodegenerative diseases.

14. A kit comprising at least one morphinan-6-one compound together with written instructions on an application or instruction leaflet or a package.
